# EUROPEAN PATENT SPECIFICATION

(11) **EP 2 663 395 B1**
(45) Date of publication and mention of the grant of the patent: **18.03.2015**
(21) Application number: 12703983.2
(22) Date of filing: 05.01.2012
(51) Int. Cl.: B01J 13/00

(54) **HYDROPHOBIC AND HYDROPHILIC AEROGELS ENCAPSULATED WITH PEG HYDROGEL VIA SURFACE INITIATED PHOTOPOLYMERIZATION**
DURCH OBERFLÄCHENINITIIERTE PHOTOPOLYMERISATION IN PEG-HYDROGELE EINGEKAPSELTE HYDROPHOBE UND HYDROPHILE AEROGELE
AÉROGELS HYDROPHOBES ET HYDROPHILES ENCAPSULÉS AVEC UN HYDROGEL DE POLYÉTHYLÈNE GLYCOL (PEG) PAR L'INTERMÉDIAIRE D'UNE PHOTO-POLYMÉRISATION AMORCÉE EN SURFACE

(30) Priority: 10.01.2011 TR 201100234
(43) Date of publication of application: 20.11.2013
(73) Proprietor: KOC Universitesi, 34450 Istanbul (TR)
(72) Inventor: GIRAY, Seda, 34450 Istanbul (TR); KIZILEL, Seda, 34450 Istanbul (TR); ERKEY, Can, 34450 Istanbul (TR)
(74) Representative: Cayli, Hülya
(86) International application number: PCT/EP2012/050116
(87) International publication number: WO 2012/095346

(56) References cited:
- WO-A2-03/057367
- US-A1- 2004 118 748
- US-A1- 2004 118 749

## Description

### Technical Field

The present invention relates to a novel composite of silica aerogel and a hydrogel and a method for the sequential formation thereof. The composite was synthesized by encapsulation of hydrophobic aerogels with PEG hydrogel via photoinitiated polymerization. The aerogel-hydrogel composite of the present invention consists of two layers: the outer hydrogel layer is hydrophilic, whereas the inner aerogel core is hydrophobic.

The aim of the present invention is to control the drug release in different ways by modifying the network of hydrogels as well as structurally adjusting hydrophobic characteristics of the aerogel. The novel composite would especially be attractive for drug or protein delivery, biomaterials and other situations where unique properties of both aerogels and hydrogels would be used.

### Background of the Invention

Silica aerogels are sol-gel derived materials with high surface areas, high pore volumes and low densities. These materials are produced by supercritical drying of the gels obtained via hydrolysis and condensation of a silicon alkoxide precursor such as tetraethylorthosilicate (TEOS) in a solvent. The properties of silica aerogels can be tailored by manipulation of reaction conditions and reactant concentrations during their synthesis and they can be produced as monoliths in any shape. As a result of such favorable properties, silica aerogels have been under investigation for use in various applications such as thermal insulation, dust collectors, glazing windows and particle detectors since their discovery in the 1930s. The tunable surface and pore properties of porous silica aerogels make them promising candidates for the development of novel drug delivery devices. For example, a drug adsorbed on a hydrophilic silica aerogel can be released much faster than from its crystalline form. The loading and the release rate of the drug in the aerogel matrix can be controlled by the hydrophobicity of the aerogel surface.

Another important class of materials in pharmaceutics, biotechnology and medicine is hydrogels. They have been prepared for use as drug carriers for the release of drugs, peptides and proteins due to their three dimensional, hydrophilic networks. For example, polyethylene glycol (PEG) can be chemically crosslinked into hydrogels and used as reservoir for the controlled delivery of smaller molecular weight drugs. PEG hydrogel has received significant attention, especially because of its non-toxic, non-immunogenic and hydrophilic properties. Previous studies investigated the kinetics of PEG hydrogel formation, and diffusion of various drugs and/or proteins from these PEG hydrogels. Hydrogels can be designed to be responsive to properties such as pH, temperature, concentration of a metabolite or electric field which may be utilized for different applications.

Prior art does not contain any similar studies wherein aerogel is coated with hydrogel and the resulting composite is used as a drug carrier. However, there are some studies wherein aerogel and hydrogel are used as drug carriers separately. In these studies, different monomers and hydrogels which are designed to be responsive to a specific change in the medium are used.

WO 03/057367 A2 discloses a process for making a composition comprising forming a cured hydrogel and removing a portion of the liquid medium from the cured hydrogel to form a dried silica aerogel.

In the present invention, integration of aerogel and hydrogel within a single hybrid structure is provided and a novel composite is obtained. The advantage of the novel composite of the present invention is that it controls the drug release in two ways and also provides the "consecutive" drug release. The control mechanisms thereof are as follows:
1. tuning of the hydrophobicity of aerogel
2. coating of aerogel with PEG hydrogel

The novel aerogel-hydrogel composite consists of two layers: the outer hydrogel layer is hydrophilic, whereas the inner aerogel core is hydrophobic. This two-layer composite may allow for the loading of two drugs having hydrophilic and hydrophobic properties in one single structure without contact with each other. These layers provide the controlled release of the active pharmaceutical ingredients. By changing aerogel and hydrogel properties, drug release rate can also be controlled.

One other advantage of the present invention is the possibility of the design of the outer hydrogel layer to be degradable such that it may degrade as a result of the exposure to environmental responses such pH, temperature change. This may provide an additional control on the release of protein or drug adsorbed within the inner aerogel core, and at the same time allows faster release of the drug loaded within the outer hydrogel layer. Traditionally used drug pills in pharmaceutics contain crystalline chemicals which do not provide any control on the release of therapeutic drug.

### Brief Description of the Invention

A feature of the present invention is to provide a composite of silica aerogel and PEG hydrogel.

Another feature of the present invention is to provide a novel composite synthesized by encapsulation of hydrophobic aerogels within PEG hydrogel via surface initiated photopolymerization.

Another feature of the present invention is the sequential formation of silica aerogel and hydrogel composite. The present invention relates to a process for the preparation of silica aerogel and hydrogel composite.

In the present invention, it is also disclosed a novel method for the sequential formation of silica aerogel and hydrogel composite.

The composite of the present invention was synthesized by encapsulation of hydrophobic aerogels with PEG hydrogel via photoinitiated polymerization. Disks of aerogels were synthesized by the two step sol-gel method using tetraethylortosilicate (TEOS) as the silica precursor. HCl and NH₄OH were used as hydrolysis and condensation catalysts respectively. After the gels were aged in ethanol, the alcogels were then contacted with a solution of eosin-Y, a photoinitiator, dissolved in ethanol. The adsorption of eosin-Y on the surface of alcogel led to a reddish transparent composite of silica aerogel with eosin-Y. The alcogels with eosin-Y were subsequently dried by supercritical CO₂ at 313 K and 10.3 MPa. Eosin functionalized silica aerogels was rendered hydrophobic using hexamethyldisilazane (HMDS) as the surface modification agent, and scCO₂ as solvent at 20.68 MPa and 333.2 K. The effects of HMDS concentration in the fluid phase and the reaction time were investigated and the contact angles were found to be 130° at different conditions. The hydrophobic aerogels were dipped into a PEG diacrylate prepolymer solution, and photopolymerization was carried out using visible light (514 nm). The hydrogel coating around the hydrophobic aerogel was only restricted to the external surface of the monolithic disks, since the water based prepolymer solution did not penetrate into the hydrophobic aerogel structure. BET surface area and pore size distribution measurements were done for both non-coated and coated aerogel. The data show that both hydrogel encapsulation and eosin-Y loading did not affect the pore structure of the aerogel.

The present invention would especially be attractive for drug and protein release, biomaterials and other situations where unique properties of both aerogels and hydrogels would be used. The present invention also relates to the release of drugs or proteins with different hydrophobicities within the composite structure of the present invention.

### Brief Description of the Figures

Figure 1: Schematic representation of the photoinitiation process
Figure 2: Schematic representation of the overall synthesis
Figure 3: Image of aerogels and hydrogels
Figure 4: Effect of eosin loading and surface modification on nitrogen adsorption and on pore size distribution

### Reference List

2.a: Use of HCl and NH₄OH respectively as hydrolysis and condensation catalysts
2.b: Aging the alcogels in ethanol-water (50 wt. %) solution at 323 K for 1 day and in ethanol solution at room temperature for 3 days
2.c: Formation of a reddish transparent composite of silica alcogel with eosin-Y
2.d: supercritical drying with CO₂(scCO₂) at 313 K and 10.3 MPa
2.e: Rendering hydrophilic and eosin functionalized aerogels hydrophobic using supercritical fluid deposition technique
2.f: Carrying out the photopolymerization
2.g: Image of the PEG hydrogel coated hydrophobic aerogels
3.a: Image of the pure aerogel,
3.b: Image of the Eosin doped hydrophilic aerogel,
3.c: Image of water droplet on the Eosin doped hydrophobic aerogel
3.d: Image of the hydrogel coated hydrophobic aerogel.
4.a: Effect of eosin loading and surface modification on nitrogen adsorption
4.b: Effect of eosin loading and surface modification on pore size distribution

### Detailed Description of the Invention

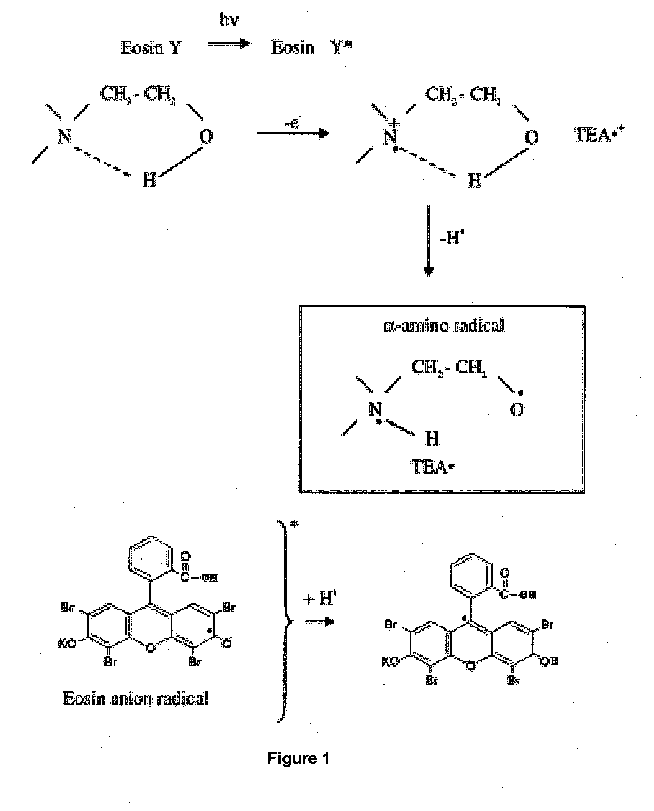

In the present invention, a novel composite material was synthesized by encapsulation of hydrophobic aerogels within PEG hydrogel via surface initiated photopolymerization. Immobilized initiator on the surface of the aerogel started the formation of PEG diacrylate hydrogels on the surface. Eosin was used as the photoinitiator because of its spectral properties that perfectly suit its use as an initiating system for an argon ion laser. In the presence of an electron donor such as triethanolamine (TEA), which acts as a co-initiator, eosin initiates acrylate polymerization when irradiate. It is generally accepted that polymerization occurs as a result of the formation of the free radicals originating from triethanolamine. The photoinitiation mechanism, as described in figure 1, involves irradiation with green light, as a result of which, eosin is excited to the triplet state. Subsequently, electron transfer from triethanolamine to the excited triplet state of the eosin dye produces an eosin anion radical and a triethanolamine cation radical. This is followed by proton loss from the triethanolamine cation radical (TEA^{.+}), resulting in a neutral α-amino radical (TEA), which is generally assumed to initiate free-radical polymerization. Simultaneously, the proton released from the triethanolamine cation radical is transferred to the eosin anion radical, yielding a neutral eosin radical.

The present invention relates to a technique to synthesize a novel composite of hydrophobic aerogel and hydrogel, which was formed as a result of encapsulation of hydrophobic aerogel within PEG hydrogel via surface initiated photopolymerization. The results showed that the hydrogel encapsulation did not alter the porous structure of the aerogel.

The novel composite of the present invention will have an important role in pharmaceutics due to its unique structure which may allow the loading of two different drugs and sequential drug release. Due to the bilayer structure of the composite, the release sequence of the drugs can also be adjusted such that hydrophilic drug located in the outer layer will dissolve first, and hydrophobic drug loaded within the hydrophobic aerogel will release subsequently. It is also possible to design a degradable PEG hydrogel layer, such that the network structure can be broken down through biological processes such as enzymatic digestion or as a result of change in pH which may enhance the release of the drug loaded within the aerogel.

In the present invention, combining the aerogel and hydrogel into the single hybride structure is provided and a novel composite is obtained. The advantage of the novel composite of the present invention is that it controls the drug release in two ways and also provides the "consecutive" drug release. The control mechanisms thereof are as follows:
1) tuning of the hydrophobicity of aerogel
2) coating of aerogel with PEG hydrogel

The novel aerogel-hydrogel composite consists of two layers: the outer hydrogel layer is hydrophilic, whereas the inner aerogel core is hydrophobic. According to the "consecutive" drug release, the drug loaded on the hydrogel on the outer layer is released by diffusion and subsequently the release of the drug loaded on the aerogel of the inner layer occurs.

The novel composite would especially be attractive for drug and protein release, biomaterials and other situations where unique properties of both aerogels and hydrogels would be beneficial.

The novel composite of the subject of the invention can be utilized to provide controlled release of various drugs by pharmaceutical companies. This drug carrier may be used as a new drug form or other type dosage forms in medicine and pharmaceutics. For the diseases such as cancer, the requirement to take many drugs for patients may be eliminated with the use of a carrier such as the one introduced in the present invention. One of the most common drug-induced injuries is irritation of the lining of the stomach caused by nonsteroidal anti-inflammatory drugs (NSAIDs). To prevent that irritation, patients first must take a drug to protect the stomach lining. In such cases, the bilayer structure of the composite of the present invention may lead to the release of primary drug protect the stomach and then let the release of main drug chemical.

### Working Examples

In the present invention, for the synthesis of silica aerogels, tetraethylorthosilicate (TEOS) (98.0 %) and ammonium hydroxide (NH₄OH) (2.0M in ethanol) were purchased from Aldrich, hydrochloric acid (HCl) was purchased from Riedel-de Haen (37%). Ethanol was obtained from Merck (99.9%). For the modification, hexamethyldisilazane (HMDS) was obtained from Alfa Aesar (98%). Carbon dioxide (99.998 %) was purchased from Messer Aligaz. For the hydrogel formation, Eosin Y (98%), 1-vinyl 2-pyrrolidinone (99+%), poly(ethylene glycol) diacrylate (PEG-DA) (MW ¼ 575 Da) were obtained from Aldrich. Triethanolamine (>99.5%) was obtained from Fluka. All chemicals were used as-received.

### Procedure of synthesis of silica aerogel and modifications

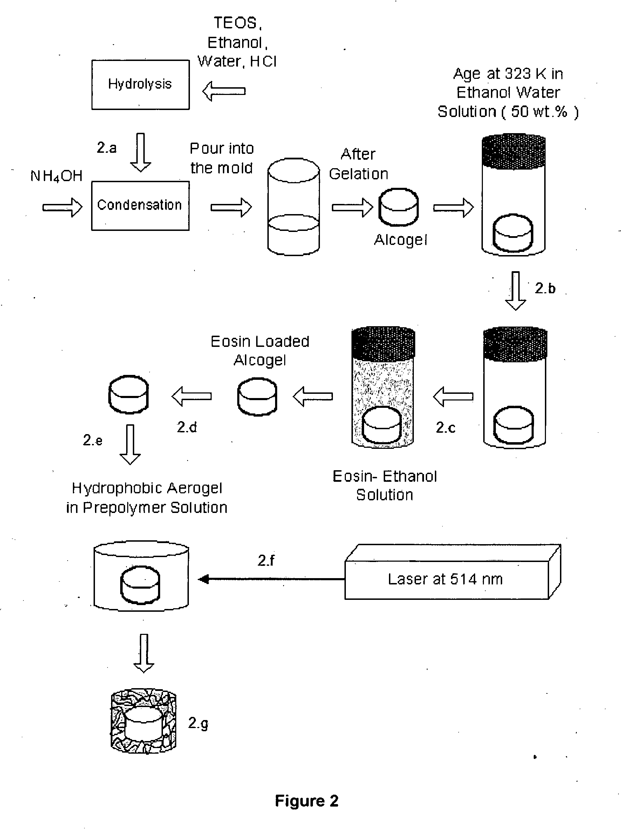

Disks of aerogels with a diameter of 13.7 mm and a height of 3.3 mm were synthesized by the two step sol-gel method using TEOS as the silica precursor. HCl and NH₄OH were used as hydrolysis and condensation catalysts respectively (Fig. 2-a). The overall molar ratio of TEOS: Water: HCl: NH₄OH were kept constant at 1: 4: 2.44x10⁻³: 2x10⁻² respectively. The alcogels were aged in ethanol-water (50 wt. %) solution at 323 K for 1 day and in ethanol solution at room temperature for 3 days (Fig. 2-b). The aim of the aging step was to improve the mechanical strength of the alcogels. After aging step, they were contacted with 2mM eosin-Y, a photoinitiator, in ethanol solution. The adsorption of eosin-Y on the surface of alcogel led to a reddish transparent composite of silica alcogel with eosin-Y (Fig. 2-c). The silica alcogels with eosin-Y were subsequently dried by supercritical CO₂ (scCO₂) at 313 K and 10.3 MPa (Fig. 2-d).

The hydrophilic and eosin functionalized aerogels, formed in steps a through e, were rendered hydrophobic using supercritical fluid deposition technique. Hexamethyldisilazane (HMDS) was used as the surface modification agent, and supercritical carbon dioxide (scCO₂) as solvent at 20.68 MPa and 333.2 K (Fig. 2-e). By replacing the hydrogen atoms in the surface silanol groups by a hydrolytically stable organofunctional group (e.g. Si-(CH₃)₃), hydrophobic aerogels were obtained. Finally, eosin loaded hydrophobic aerogels were immersed in PEG-diacrylate polymer solution and photopolymerization was carried out using visible light (514 nm) for 3 min for each surface of the aerogels (Fig. 2-f). The hydrogel precursor was prepared with concentrations of 225 mM triethanolamine, 25% (w/w) PEG diacrylate (MW = 575 Da), and 37mM 1-vinyl-2-pyrrolidinone (NVP). The solution was adjusted to pH 8 using HCl. Prepolymer solution was sterilized using a 0.2 µm syringe Teflon filter. This step resulted in the formation of a crosslinked thin PEG hydrogel coating through surface-initiated polymerization around the hydrophobic aerogels. (Fig. 2-g)

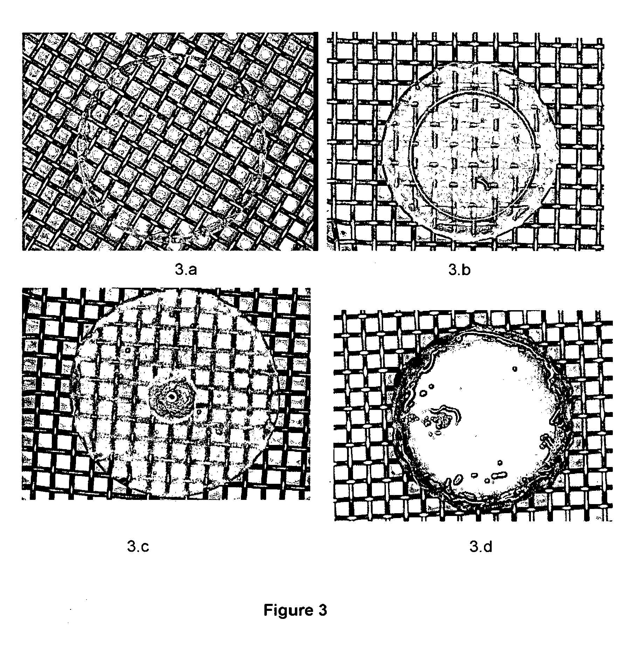

The colorless transparent aerogel obtained a red color due to the presence of eosin within the aerogel structure. Eosin molecules were homogeneously distributed throughout the aerogel (Figures 3-a and 3-b). After surface modification step, hydrophobicity of the aerogel was verified by placing a water droplet and measuring the contact angle on the surface of the aerogel (Fig. 3-c). The contact angle for the eosin modified hydrophobic aerogel was found to be 130°. Figure 3-d shows the image of a PEG hydrogel coated eosin functionalized hydrophobic aerogel. The thickness of the hydrogel coating was approximately 0.3 mm.

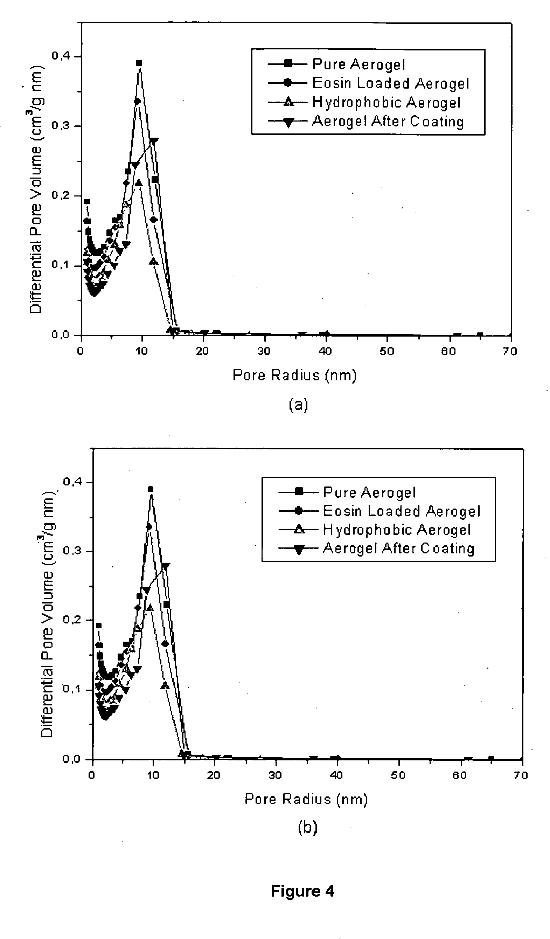

The effects of the eosin loading and the surface modification step on the pore structure of the aerogel were investigated with the nitrogen adsorption analysis by Micromeritics ASAP 2020 surface analyzer. As seen in Table 1, the presence of eosin on the aerogel surface caused the BET surface area to decrease slightly with no appreciable changes in the average pore diameter. Further modification of the eosin functionalized aerogel surface with HMDS decreased the total pore volume and surface area and also increased the cumulative pore size slightly. This can perhaps be attributed to the presence of some bottleneck type pores which are blocked by Si-(CH₃)₃ groups. The adsorption isotherms and pore size distributions of modified aerogels are compared in Figures 4-a and 4-b. All samples exhibited similar pore size distribution and type H1 isotherm which indicates that the materials consist of compacts agglomerates of approximately uniform spheres of silica and such a network is not disrupted by eosin loading, surface modification, and PEG hydrogel coating.

BET surface area and pore size distribution measurements were carried out for both non-coated and coated aerogels (Table 1). The data showed that both hydrogel encapsulation and eosin-Y loading did not affect the pore structure of aerogel. Also, the isotherms and pore distributions of the two samples were nearly identical which indicate that the hydrogel coating was only restricted to the external surface of the monolithic disks and the water based prepolymer solution did not diffuse into the hydrophobic aerogel structure.

**Table 1: Properties of Aerogel Composites After Each Step in Synthesis**

| | BET Surface Area | BJH Desorption Cumulative Pore Volume | BJH Desorption Average Pore Radius |
|---|---|---|---|
| Pure Aerogel | 926 m²/g | 2.9 cm³/g | 5.9 nm |
| Eosin Loaded Aerogel | 820 m²/g | 2.5 cm³/g | 6.0 nm |
| After Surface Modification | 528 m²/g | 2.1 cm³/g | 6.4 nm |
| After Hydrogel Coating | 529 m²/g | 2.2 cm³/g | 6.7 nm |

## Claims

1. A composite consisting of a hydrophobic and eosin functionalized silica aerogel core encapsulated by an outer PEG hydrogel layer through surface-initiated photopolymerization.

2. A process for the production of a composite according to claim 1 **characterized in that** the process comprises formation of eosin functionalized silica aerogel and the coating of PEG hydrogel around said aerogels through surface-initiated photopolymerization.

3. A process for the production of a composite according to claim 2 **characterized in that** the formation of said eosin functionalized silica aerogel comprises the following steps:
a. Disks of aerogels with a diameter of 13.7 mm and a height of 3.3 mm were synthesized by the two step sol-gel method using tetraethylorthosilicate as the silica precursor, HCl and NH₄OH to obtain alcogels,
b. The alcogels were aged in ethanol-water solution at 323 K for 1 day and in ethanol solution at room temperature for 3 days,
c. The aged alcogels were contacted with 2mM eosin-Y, a photoinitiator, in ethanol solution, and
d. The alcogels with eosin-Y were subsequently dried by supercritical CO₂ at 313 K and 10.3 MPa.

4. A process for the production of a composite according to claim 2 **characterized in that** the coating of hydrogel around eosin functionalized silica aerogel through surface-initiated photopolymerization comprises the following steps:
a. The obtained hydrophilic and eosin functionalized aerogel is reacted with hexamethyldisilazane as the surface modification agent and scCO₂ as solvent at 20.68 MPa and 333.2 K to obtain hydrophobic aerogels,
b. Eosin loaded hydrophobic aerogels were immersed in PEG-diacrylate polymer solution and photopolymerization was carried out using visible light of 514 nm for 3 min for each surface of the aerogels,
c. PEG hydrogel prepolymer solutions were filter sterilized using a 0.2 µm syringe Teflon filter, and
d. PEG hydrogel coating is formed around the hydrophobic aerogel through surface-initiated polymerization.

## Patentansprüche

1. Komposit bestehend aus einem hydrophoben und Eosin- funktionalisiertem Silica-Aerogelkern, eingekapselt von einer äußeren PEG-Hydrogelschicht, durch oberflächeninitiierte Photopolymerisation.

2. Verfahren zu Herstellung eines Komposits nach Anspruch 1, **dadurch gekennzeichnet, dass** das Verfahren die Bildung von Eosin-funktionalisiertem Silica-Aerogel und die Beschichtung mit PEG-Hydrogel um die Aerogele, durch oberflächeninitiierte Photopolymerisation, beinhaltet.

3. Verfahren zur Herstellung eines Komposits nach Anspruch 2, **dadurch gekennzeichnet, dass** die Bildung des Eosin-funktionalisiertem Silica-Aerogel folgende Schritte umfasst:
**a.** Synthese von Aerogel-Scheiben mit einem Durchmesser von 13,7 mm und einer Höhe von 3,3 mm durch das zweistufige Sol-Gel-Verfahren mit Tetraethylorthosilicat als Silicapräkursor, HCl und NH₄OH zur Bildung von Alkogelen,
**b.** Altern der Alkogele in einem Ethanol-Wasser-Gemisch bei 323 K für einen Tag und in einer Ethanollösung bei Raumtemperatur für drei Tage,
**c.** in Kontakt bringen der gealterten Alkogele mit 2 mM Eosin-Y, einem Photoinitiator, in Ethanollösung und
**d.** Trocknung der Alkogele mit Eosin-Y durch überkritisches CO₂ bei 313 K und 10,3 MPa.

4. Verfahren zur Herstellung eines Komposits nach Anspruch 2, **dadurch gekennzeichnet, dass** die Beschichtung mit Hydrogel um das Eosin- funktionalisierte Silica-Aerogel, durch oberflächeninitiierte Photopolymerisation, die folgende Schritte umfasst:
**a.** Reaktion des erhaltenen hydrophilen und Eosin-funktionalisiertem Aerogel mit Hexamethyldisilazan als Oberflächenmodifikationsmittel und überkritischem CO2 (scCO2) als Lösungsmittel bei 20,68 MPa und 333,2 K zur Bildung von hydrophoben Aerogelen,
**b.** Einbringen der Eosin-beladenen hydrophoben Aerogele in PEG- Diacrylat-Polymerlösung und Photopolymerisation unter Verwendung von sichtbarem Licht bei einer Wellenlänge von 514 nm für drei Minuten für jede Seite des Aerogels,
**c.** Filtersterilisation der PEG-Hydrogelprepolymerlösungen durch einen 0,2 µm Teflon-Spritzenfilter und
**d.** Bildung einer PEG-Hydrogelbeschichtung um das hydrophobe Aerogel durch oberflächeninitiierte Polymerisation.

## Revendications

1. Composite constitué d'un coeur d'aérogel de silice hydrophobe à fonctionnalités éosine encapsulé par une couche extérieure d'hydrogel de PEG par photopolymérisation amorcée en surface.

2. Procédé de production d'un composite selon la revendication 1, **caractérisé en ce que** le procédé comprend la formation d'un aérogel de silice à fonctionnalités éosine et le revêtement de l'hydrogel de PEG autour desdits aérogels par photopolymérisation amorcée en surface.

3. Procédé de production d'un composite selon la revendication 2, **caractérisé en ce que** la formation dudit aérogel de silice à fonctionnalités éosine comprend les étapes suivantes :
a. synthèse de disques d'aérogels d'un diamètre de 13,7 mm et d'une hauteur de 3,3 mm par le procédé sol-gel à deux étapes en utilisant du tétraéthylorthosilicate en tant que précurseur de silice, du HCl et du NH₄OH pour obtenir des alcogels,
b. vieillissement des alcogels dans une solution aqueuse d'éthanol à 323 K pendant 1 jour et dans une solution d'éthanol à température ambiante pendant 3 jours ;
c. mise en contact des alcogels soumis à vieillissement avec de l'éosine Y 2 mM, un photoinitiateur, dans une solution d'éthanol, et
d. séchage subséquent des alcogels avec l'éosine Y par CO₂ supercritique à 313 K et 10,3 MPa.

4. Procédé de production d'un composite selon la revendication 2, **caractérisé en ce que** le revêtement de l'hydrogel autour de l'aérogel de silice à fonctionnalités éosine par photopolymérisation amorcée en surface comprend les étapes suivantes :
a. mise en réaction de l'aérogel hydrophile à fonctionnalités éosine obtenu avec de l'hexaméthyldisilazane en tant qu'agent de modification de surface et du scCO₂ en tant que solvant à 20,68 MPa et 333,2 K pour obtenir des aérogels hydrophobes,
b. immersion des aérogels hydrophobes chargés d'éosine dans une solution de polymère de diacrylate de PEG et réalisation de la photopolymérisation en utilisant une lumière visible de 514 nm pendant 3 minutes pour chaque surface des aérogels,
c. stérilisation par filtration des solutions de prépolymère d'hydrogel de PEG en utilisant un filtre Teflon pour seringue de 0,2 µm, et
d. formation du revêtement d'hydrogel de PEG autour de l'aérogel hydrophobe par polymérisation amorcée en surface.
